(19) Europäisches Patentamt
European Patent Office
Office européen des brevets

(11) EP 0 683 236 B1

(12) **FASCICULE DE BREVET EUROPEEN**

(45) Date de publication et mention de la délivrance du brevet:
**09.08.2000 Bulletin 2000/32**

(51) Int Cl.[7]: **C12P 41/00**, C07C 62/14, C12P 7/40

(21) Numéro de dépôt: **95400878.5**

(22) Date de dépôt: **20.04.1995**

(54) **Procédé enzymatique pour la préparation de dérivés tétraliniques optiquement actifs**

Enzymatisches Verfahren zur Herstellung von optisch-aktiven Tetralin-Derivaten

Enzymatic process for the preparation of optical active tetralin-derivatives

(84) Etats contractants désignés:
**AT BE CH DE DK ES FR GB GR IE IT LI LU MC NL PT SE**
Etats d'extension désignés:
**LT SI**

(30) Priorité: **21.04.1994 EP 94400863**

(43) Date de publication de la demande:
**22.11.1995 Bulletin 1995/47**

(73) Titulaires:
- **Sanofi-Synthélabo**
  **75013 Paris (FR)**
  Etats contractants désignés:
  **BE CH DE DK ES FR GB GR IE LI LU MC NL PT SE AT**
- **SANOFI WINTHROP S.p.A.**
  **20137 Milano (IT)**
  Etats contractants désignés:
  **IT**

(72) Inventeurs:
- **Cecchi, Roberto**
  **I-20075 Lodi (Milano) (IT)**
- **Barzaghi, Laura**
  **I-20052 Monza (Milano) (IT)**
- **Guzzi, Umberto**
  **I-20100 Milano (IT)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 300 404**
**EP-A- 0 436 435**
**DE-A- 3 345 660**

EP 0 683 236 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention concerne un procédé pour la préparation de dérivés optiquement actifs de la tétraline.

Dans les dix dernières années, l'application des enzymes dans la synthèse enantiosélective a été amplement étudiée (Klibanov, Acc. Chem. Res., 23 : 114, 1990). Plus particulièrement, l'enzyme lipase a été utilisée dans des estérifications et transestérifications asymétriques, notamment dans la préparation d'alcools, esters et acides.

De nombreux dérivés à structure tétralinique, sous forme optiquement active, sont décrits en littérature, notamment en tant qu'intermédiaires de synthèse (EP-A-436435, EP-B-347313, EP-A-211721, DE 2803582, EP-A-334538) ou comme outils de laboratoire dans les essais pharmacologiques et biochimiques. Par exemple, la 8-hydroxy-2-diisopropylaminotétraline (8-OH-DPAT) et ses enantiomères (*R*) et (*S*) sont utilisés comme produits de référence dans les tests sur la sérotonine, notamment en tant que $5\text{-}HT_{1A}$ agonistes (J. Med. Chem., 1989, 32 : 779-783; Eur. J. Med. Chem., 1991, 26: 215-220).

Plus particulièrement EP-A-436435 décrit des acides 1,2,3,4-tétrahydro-2-naphtoïques méthoxysubstitués, sous forme optiquement active. La séparation des énantioméres de ces acides à partir du racémate, lorsqu'elle est effectuée par les méthodes chimiques habituelles, est non seulement laborieuse mais aussi peu performante et les résultats, en termes de pureté énantiomérique des produits séparés, sont parfois peu satisfaisants.

La demande DE-A-33 45 660 décrit un procédé pour l'extraction de l'acide D-2-(6-méthoxynapht-2-yl)propionique à partir d'un mélange d'esters d'alkyle inférieur de l'acide L- et D-2-(6-méthoxynapht-2-yl)propionique, qui comprend les étapes suivantes :

a) hydrolyse asymétrique de l'ester L- avec une enzyme bactérienne ;
b) séparation de l'acide 2-(6-méthoxynapht-2-yl)propionique de l'ester d'alkyle inférieur de l'acide D-2-(6-méthoxynapht-2-yl)propionique; et
c) saponification dudit ester avec une esterase de foie de porc ou une esterase de *Pleurotus ostreatus.*

On a maintenant trouvé qu'en se basant sur la cinétique d'une hydrolyse enzymatique d'esters alkyliques d'acides tétralinecarboxyliques, on peut effectuer la résolution des énantiomères par une réaction très simple et performante.

Notamment, il a été trouvé qu'en soumettant un ester 1,2,3,4-tétrahydro-2-naphtoïque méthoxy-substitué à l'action de la lipase de pancréas de porc l'enzyme hydrolyse préférablement la forme (R) de l'ester, en laissant la forme (*S*) presque inaltérée, en permettant ainsi de séparer les deux formes.

La présente invention a donc pour objet un procédé de préparation des composés de formule (I) sous forme optiquement active

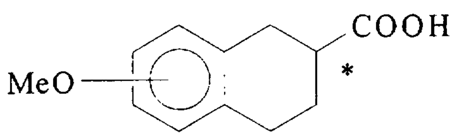

(I)

caractérisé en ce que:

(a) on soumet un ester racémique de formule

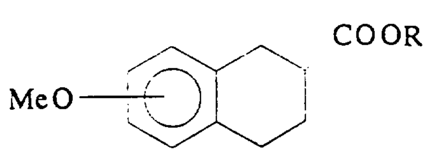

(II)

dans laquelle R est un alkyle en $C_1$-$C_3$, à une hydrolyse par la lipase de pancréas de porc ; puis
(b) lorsque environ 50 % de l'ester est hydrolysé en acide, on interrompt l'hydrolyse par inactivation de l'enzyme et on récupère l'ester de configuration (*S*) de formule

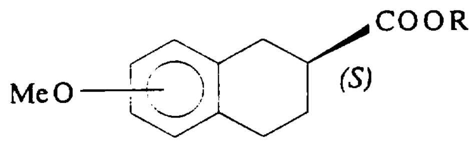

(II')

qui n'a pas réagi, enfin

(c) soit on hydrolyse ledit ester de formule (II') et on isole l'acide de configuration (*S*) de formule

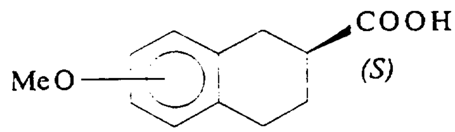

(I')

soit on isole l'acide de configuration (*R*) de formule

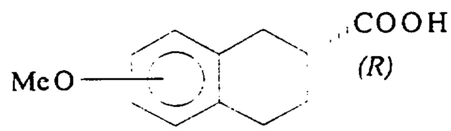

(I")

tel qu'obtenu de la réaction d'hydrolyse lipasique, après interruption de l'hydrolyse à l'étape (b).

Dans les formules (I), (II), (I'), (I") et (II') ci-dessus et dans la description ci-après, Me désigne le groupe méthyle.

Les esters de formule (II) sont décrits par exemple dans EP-A-300404, EP-A-436435, Synthesis, 9:727-9, (1983), J. Am. Chem. Soc., 104:7609-22, (1982), ou bien ils peuvent être aisément préparés par estérification des acides racémiques correspondants. Parmi ces produits de départ ceux de formule (II) où R est méthyle ou éthyle sont particulièrement préférés.

La lipase utilisée dans la réaction de l'étape (a) est la lipase de pancréas de porc (PPL de l'anglais "porcine pancreatic lipase"), qui est une enzyme vendue dans le commerce sous forme fraîche ou lyophilisée (de préférence la lipase utilisée est la lipase de pancréas de porc - Type II, Sigma L-3126). L'enzyme lipase peut éventuellement être immobilisée, par des liaisons covalentes, sur une matrice polymérique, par exemple une résine selon les techniques décrites par exemple par S. Fukuy, Enzymes Eng., 6:191-200 (1982).

La réaction d'hydrolyse énantiosélective de l'étape (a) est effectuée de préférence en milieu aqueux, en mélange avec un solvant organique miscible à l'eau et en présence d'un système tampon. Plus particulièrement, le mélange réactionnel est tamponné par un tampon à un pH d'environ 7, préparé selon des méthodes connues tel que par exemple le tampon phosphate. Ce pH correspond au pH des performances optimales de l'enzyme.

La température de réaction de l'étape (a) peut être librement choisie dans un intervalle de température dans lequel l'enzyme n'est pas desactivée, allant généralement de 0° C à 60° C, avantageusement de 5° C à 40° C, de préférence de 10° à 30° C, normalement à la température ambiante.

Le solvant organique utilisé dans l'étape (a) du procédé de l'invention est un solvant miscible à l'eau, tel que par exemple un alcool, comme le méthanol, l'éthanol, le n-propanol, l'*iso*-propanol, le n-butanol, le *sec*-butanol, l'*iso*-butanol, le *tert*-butanol; une cétone, comme l'acétone; un éther cyclique, comme le tétrahydrofurane ou le dioxane; le *tert*-butanol étant un solvant particulièrement avantageux. La quantité dudit solvant n'est pas un paramètre critique ; généralement, on utilise une quantité de solvant suffisante à dissoudre l'ester de départ.

Normalement, l'ester racémique de formule (II) est dissous dans un solvant organique miscible à l'eau et la solution obtenue est ajoutée à un tampon à pH 7. A ce mélange est ensuite ajoutée l'enzyme PPL qui catalyse l'hydrolyse sélective de l'énantiomère (*R*) de l'ester. La réaction est contrôlée par addition d'une base, notamment NaOH, pour maintenir la valeur du pH presque constante à un pH d'environ 7. La réaction est terminée lorsque l'enzyme a hydrolysé la moitié de l'ester, notamment l'isomère (*R*) de l'ester, à savoir après l'addition d'une quantité de base égale au maximum à la moitié d'équivalents-mole de l'ester de départ.

Dans l'étape (b), pour arrêter la réaction, on inactive l'enzyme, par exemple par addition d'une base, de façon à augmenter le pH de la solution jusqu'à une valeur basique, de préférence d'au moins environ 8. La base utilisée peut être inorganique, comme un hydroxyde alcalin, notamment de sodium ou de potassium, un carbonate alcalin, notamment de sodium ou de potassium ou une amine, de préférence tertiaire, telle que la triéthylamine.

L'isomère ayant la configuration (*S*) de l'ester, de formule (II'), qui n'a pas été hydrolysé par la lipase de pancréas de porc, est extrait dans un solvant organique immiscible à l'eau et isolé selon les techniques habituelles, par exemple par évaporation du solvant sous pression réduite.

La phase aqueuse résiduelle renferme l'acide libre correspondant à l'ester de départ de formule (II) de configuration absolue (*R*).

Dans l'étape (c), on procède à la séparation des deux énantiomères (*S*) et (*R*).

Pour isoler l'isomère (*S*) de formule (I'), on soumet l'ester méthylique (II') à une hydrolyse en conditions acides ou basiques. L'hydrolyse en conditions basiques correspond à une saponification effectuée selon les techniques habituelles, par exemple avec un hydroxyde alcalin tel que l'hydroxyde de sodium ou de potassium. L'acide (I') est isolé selon les techniques conventionnelles, par acidification avec un acide minéral ou organique, par exemple à l'aide de l'acide chlorhydrique ou sulfurique. L'hydrolyse en conditions acides est effectuée avec un acide tel que l'acide chlorhydrique ou sulfurique et l'énantiomère (*S*) de formule (I') est isolé par neutralisation avec une base telle que l'hydroxyde de sodium.

Pour l'obtention de l'énantiomère (*R*) de formule (I"), ledit acide, qui constitue le produit de l'hydrolyse par la lipase de l'étape (a) et qui reste dans la phase aqueuse après la séparation de l'ester (II'), est récupéré et isolé selon les méthodes conventionnelles, notamment par acidification de la solution aqueuse et filtration du précipité formé.

Lorsque l'on désire obtenir les dérivés (I") de configuration absolue (*R*), il est avantageux d'arrêter la réaction de l'étape (a) juste avant que 50% de l'ester soit hydrolysé, à savoir avant que tout l'isomère (*R*) de l'ester réagisse. Cela permet d'obtenir l'acide (*R*) de formule (I") pur, avant que la réaction parasite d'hydrolyse de l'isomère (*S*) de l'ester de formule (II) intervienne.

Si, au contraire, on souhaite obtenir les dérivés de configuration absolue (*S*), il est convenable de poursuivre l'hydrolyse un peu au-delà de la consommation de 50% de l'ester (II), de façon à être certain que tout l'isomère (*R*) est hydrolysé par l'enzyme et que tout l'ester résiduel a donc la configuration (*S*).

Ainsi, selon un aspect préféré de la présente invention, dans l'étape (b) on interrompt l'hydrolyse enzymatique juste avant que 50% du composé (II) soit hydrolysé, par addition d'hydroxyde de sodium jusqu'à pH basique, on extrait le composé (II') qui n'a pas réagi dans un solvant organique immiscible à l'eau puis on élimine la phase organique et, dans l'étape (c) on traite la phase aqueuse avec un acide minéral ou organique et on sépare l'acide de configuration (*R*) de formule (I") ainsi précipité.

Selon un autre aspect préféré de la présente invention, dans l'étape (b) on interrompt l'hydrolyse enzymatique juste après que 50% du composé (II) est hydrolysé, par addition d'hydroxyde de sodium jusqu'à pH basique, on extrait le composé (II') qui n'a pas réagi dans un solvant organique immiscible à l'eau puis on élimine la phase aqueuse, on isole l'ester (II') et, dans l'étape (c) on hydrolyse ledit ester de formule (II') et on isole l'acide de configuration (*S*) de formule (I').

Les acides isomères (*R*) et (*S*) sont obtenus par le procédé de la présente invention sous forme optiquement pure, les excès énantiomériques étant de loin supérieurs à 90%.

Les esters (II') qui peuvent être isolés sous forme extrêmement pure sont des produits nouveaux et constituent un objet ultérieur de la présente invention. Parmi ces composés, ceux de formule (II') où R représente un méthyle sont particulièrement préférés. Parmi ces derniers, ceux pour lesquels MeO est en position 6 ou 7 sont avantageux.

Les acides énantiomériques de formule (I) dans laquelle le groupe méthoxy est dans la position 8 du tétrahydronaphtalène sont des composés nouveaux, utiles en tant qu'intermédiaires dans la synthèse de la 8-OH-DPAT optiquement active.

Leur configuration absolue a été déterminée par transformation de l'acide supposé être l'isomère (R), directement obtenu par l'hydrolyse enzymatique ci-dessus, en le dérivé 8-méthoxy-2-(N-benzyl)amino 1,2,3,4-tétrahydronaphtalène optiquement actif correspondant, par une réaction stéréoconservative; le 8-méthoxy-(2*R*)-2-(N-benzyl)amino-1,2,3,4-tétrahydronaphtalène est décrit dans Acta Chem. Scand. B, 1988, 42: 231 et par la comparaison des pouvoirs rotatoires, on a pu confirmer la configuration (R) dudit acide.

Selon un aspect ultérieur, la présente invention concerne donc les énantiomères (R) et (S) de l'acide 8-méthoxy-1,2,3,4-tétrahydro-2-naphtoïque.

Les composés de formule (I') et (I") sont des intermédiaires de réaction très versatiles, utiles dans la synthèse de nombreux dérivés à structure tétralinique.

Plus particulièrement, les composés de formule (I') et (I") sont décrits en tant qu'intermédiaires dans les synthèses des amines optiquement actives de formule (III)

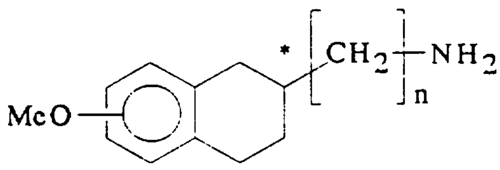

(III)

dans laquelle n est 0 ou 1 et l'astérique (*) désigne l'atome de carbone chiral dans sa forme (R) ou (S).

Le procédé pour la préparation des amines de formule (III) ci-dessus est caractérisé en ce que l'on conduit une cinétique d'hydrolyse enzymatique sur l'ester racémate de formule (II)

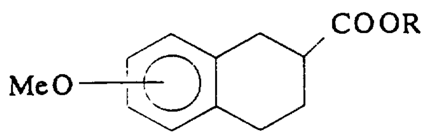

(II)

dans laquelle R est défini comme ci-dessus, à l'aide de la lipase de pancréas de porc, selon la méthode décrite dans les étapes (a) (b) et (c) ci-dessus et on transforme ensuite les acides isomères de formule (I') et (I") ainsi obtenus en amines de formule (III) selon les méthodes décrites dans EP-A-436435, notamment par la réaction de Curtius directement sur l'acide optiquement actif pour les composés de formule (III) où n=0 et par réduction de l'amide obtenue à partir de l'acide optiquement actif pour les composés où n=1.

Ainsi, selon un autre de ses aspects, la présente invention concerne un procédé pour la préparation d'une amine optiquement active de formule (III) ci-dessus dans laquelle n est 0 ou 1 et l'astérique (*) désigne l'atome de carbone chiral dans sa forme (R) ou (S), et de ses sels, caractérisé en ce que

(a) on soumet un ester racémique de formule

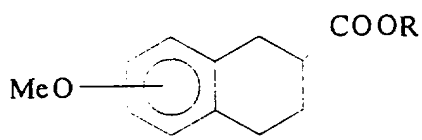

(II)

dans laquelle R est un alkyle en $C_1$-$C_3$, à une hydrolyse par la lipase de pancréas de porc ; puis

(b) lorsque environ 50 % de l'ester est hydrolysé en acide, on interrompt l'hydrolyse par inactivation de l'enzyme et on récupère l'ester de configuration (*S*) de formule

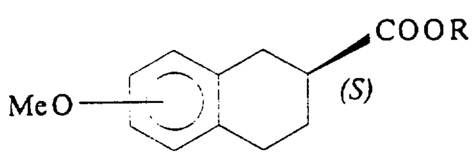

(II')

qui n'a pas réagi, puis

(c) soit on hydrolyse ledit ester de formule (II') et on isole l'acide de configuration (*S*) de formule

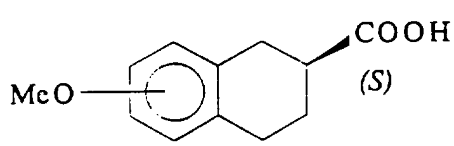

(I')

soit on isole l'acide de configuration (*R*) de formule

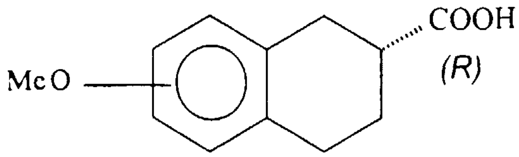

(I")

tel qu'obtenu de la réaction d'hydrolyse lipasique, après interruption de l'hydrolyse à l'étape (b), enfin
(d) soit on soumet les acides isomères à la réaction avec un azoture selon la réaction de Curtius pour isoler une amine de formule (III) où n est 0, soit on transforme les acides isomères (I') et (I") ou l'ester (II') en l'amide correspondant et on réduit ce dernier pour isoler une amine de formule (III) où n est 1; lesdites amines étant isolées sous forme de base libre ou d'un de leurs sels d'addition d'acide, ou transformées en un de leurs sels d'addition d'acides.

La réaction de Curtius de l'étape (d) peut être soit du type classique, par formation de l'azide, sa décomposition en isocyanate et l'hydrolyse de ce dernier en amine, soit du type modifié, en utilisant du diphénylphosphorylazide en présence de *tert*-butanol et hydrolyse de l'intermédiaire *tert*-butoxy carbonylamino en amine, selon les techniques décrites dans la littérature.
Ces réactions se produisent avec le maintien de la stéréoconfiguration de l'atome de carbone asymétrique.
Evidemment, lorsque l'on veut obtenir les amines de formule (III) à configuration absolue (*S*), on utilisera comme acide de départ celui de formule (I') ou son ester (II'); si l'on veut obtenir les amines de formule (III) à configuration absolue (*R*), on utilisera l'acide de formule (I") en tant que produit de départ.
Les exemples qui suivent illustrent mieux l'invention.
Les excès énantiomériques (e.e) ont été calculés sur la base des données de HPLC, dont l'analyse est effectuée dans les conditions suivantes:

(a) - Colonne : CHIRALCEL OD,

- phase mobile : mélange hexane/acide trifluoroacétique = 100/1.
- λ 280 nm

Temps de rétention (min.):

- flux : 1,0 ml/min.
- acide 6-méthoxy-1,2,3,4-tétrahydro-(2R)-2-naphtoïque = 20,3
- acide 6-méthoxy-1,2,3,4-tétrahydro-(2S)-2-naphtoïque = 23,5

Temps de rétention (min.):

- flux : 1,5 ml/min.
- acide 7-méthoxy - 1,2,3,4-tétrahydro-(2R)-2-naphtoïque = 19,2
- acide 7-méthoxy - 1,2,3,4-tétrahydro-(2S)-2-naphtoïque = 16,8

(b) - Colonne : CHIRALCEL OD,

- phase mobile : mélange hexane/isopropanol = 100/2.
- flux : 0,5 ml/min.
- λ 280 nm

Temps de rétention (min.):

- méthyl 7-méthoxy- 1,2,3,4-tétrahydro-(2R)-2-naphtoate =19,5
- méthyl 7-méthoxy- 1,2,3,4-tétrahydro-(2S)-2-naphtoate =22,6

(c) - Colonne : CHIRALCEL OD,

- phase mobile : mélange hexane/isopropanol = 85/5
- flux : 1 ml/min.

- λ 280 nm

Temps de rétention (min.):

- 6-méthoxy-1,2,3,4-tétrahydronaphtalen-(2R)-2-carboxamide = 8,96

(d) - Colonne : CHIRALCEL OD,

- phase mobile : mélange hexane/isopropanol/acide trifluoroacétique = 95/5/1
- flux : 1,3 ml/min.
- λ 280 nm

Temps de rétention (min.):

- acide 8-méthoxy-1,2,3,4-tétrahydronaphtalen-(2S)-2-napthoïque = 19,6
- méthyl-8-méthoxy-1,2,3,4-tétrahydronaphtalen-(2S)-2-napthoate = 19,2
- acide 8-méthoxy-1,2,3,4-tétrahydronaphtalen-(2R)-2-napthoïque = 10,7
- méthyl-8-méthoxy-1,2,3,4-tétrahydronaphtalen-(2R)-2-napthoate = 7,8

(e) - Colonne : CHIRALCEL OD,

- phase mobile : mélange hexane/isopropanol/acide trifluoroacétique = 95/5/1
- flux : 0,5 ml/min.
- λ 280 nm

Temps de rétention (min.):

- méthyl 6-méthoxy-1,2,3,4-tétrahydro-(2R)-2-naphtoate = 12,95
- méthyl 6-méthoxy - 1,2,3,4-tétrahydro-(2S)-2-naphtoate =14,3
- acide 5-méthoxy-1,2,3,4-tétrahydronaphtalen-(2S)-2-napthoïque = 19,3
- méthyl-5-méthoxy-1,2,3,4-tétrahydronaphtalen-(2S)-2-napthoate = 13,9
- acide 5-méthoxy-1,2,3,4-tétrahydronaphtalen-(2R)-2-napthoïque = 16,7
- méthyl-5-méthoxy-1,2,3,4-tétrahydronaphtalen-(2R)-2-napthoate = 12,5

La formule pour calculer les excès énantiomériques est la suivante:

$$e.e. = \frac{A1 - A2}{A1 + A2}$$

dans laquelle A1 et A2 représentent les aires correspondantes aux deux isomères (*R*) et (*S*) obtenues par l'analyse HPLC.

La solution tampon utilisée dans les exemples qui suivent est un tampon phosphate commercialisé par la société Merck sous le code *Merck 9439*.

## EXEMPLE 1

**Acide 6-méthoxy-1,2,3,4-tétrahydro-(2R)-2-naphtoïque.**

On dissout 3 g (0,0136 mole) de méthyl 6-méthoxy-1,2,3,4-tétrahydro-2-naphtoate dans 100 ml de *tert*-butanol et on ajoute à la solution ainsi préparée 300 ml de tampon phosphate à pH 7. La valeur du pH de la solution, qui augmente jusqu'à 7,3 - 7,5, est ramenée à 7,1 par addition de HCl 1N. On ajoute au mélange 1,5 g d'enzyme PPL Sigma (indiquée comme "porcine pancreatic lipase" type II, crude, Sigma L-3126 - 50 U/mg using triacetine). Au fur et à mesure que la réaction s'effectue, le pH tend à baisser mais on le maintient constant par addition d'une solution 0,25 N de NaOH en contrôlant le pH par un appareil automatique de titration. Après 4-5 heures, lorsque environ 22 ml de NaOH 0,25 N ont été consommés, on ajoute à la solution du bicarbonate de sodium jusqu'à pH 8, pour arrêter la réaction par inactivation de l'enzyme. On extrait dans de l'éther éthylique et on sépare les deux phases. La phase organique contient l'ester, principalement de configuration (*S*) qui n'a pas réagi et qui peut être récupéré comme indiqué dans l'Exemple 2 ci-dessous. On acidifie la phase aqueuse à l'aide d'acide sulfurique concentré et on filtre le précipité formé. On obtient ainsi 1,16 g de l'acide indiqué en titre qu'on cristallise dans l'acétate d'éthyle. P.f. 130-131°C; $[\alpha]_D^{20}$

= +57,8° (c = 1,4%, $CHCl_3$); e.e. 93,7 % (HPLC effectuée dans les conditions (a)). Ce produit est plus pur que celui décrit dans EP-A-436 435, préparation (O) (i).
Deux autres préparations effectuées dans les mêmes conditions ont donné le même produit ayant un e.e. de 92% et 93,1 % (HPLC effectuée dans les conditions (a)).

**EXEMPLE 2**

**Acide 6-méthoxy-1,2,3,4-tétrahydro-(2S)-2-naphtoïque**

La phase organique résiduelle de l'Exemple 1 est séchée sur du sulfate de sodium et évaporée sous pression réduite et on distille ensuite l'ester (Eb = 128-132°C à 0,1 mbar) contenant principalement l'isomère (*S*) (e.e. 68,9 % ; HPLC effectuée dans les conditions (a)). On dissout 5 g (0,022 mole) de l'ester ainsi recupéré dans 175 ml de *tert*-butanol; on ajoute à la solution ainsi obtenue 500 ml de tampon phosphate à pH 7. On porte à pH 7,04 par addition d'acide sulfurique à 5% et on ajoute 2,5 g de PPL Sigma (indiquée comme "porcine pancreatic lipase" type II, crude, Sigma L-3126 - 39 U/mg using triacetine). Au fur et à mesure que la réaction s'effectue, le pH baisse; on le maintient constant par addition d'une solution 0,25 N de NaOH, en utilisant un appareil automatique de titration. Lorsque 12,5 ml de NaOH 0,25 N ont été consommés, on ajoute du bicarbonate de sodium jusqu'à pH = 8 et on extrait à l'éther éthylique puis on sépare les deux phases, on élimine la phase aqueuse contenant l'acide 6-méthoxy-1,2,3,4-tétrahydro-(2R)-2-naphtoïque qui peut être récupéré, on sèche la phase organique sur du sulfate de sodium et on évapore le solvant sous pression réduite. On obtient 4,73 g de méthyl 6-méthoxy-1,2,3,4-tétrahydro-(2S)-2-naphtoate (e.e. 92% - HPLC effectuée dans les conditions (e)). On traite l'isomère (*S*) de l'ester ainsi obtenu avec une solution de NaOH, on acidifie à l'aide d'acide chlorhydrique 1N et on obtient 3,2 g de l'acide indiqué en titre qu'on cristallise dans de l'acétate d'éthyle. P.f. 130-131°C; e.e. 93% (HPLC effectuée dans les conditions (a)). Ce produit est plus pur que celui décrit dans EP-A-436 435, préparation (Q) (i).
Une autre préparation effectuée selon les mêmes conditions a donné le produit du titre ayant un e.e. de 96,6%; $[\alpha]_D^{20}$ = - 62,2° (c =1,4 %, $CHCl_3$).

**EXEMPLE 3**

**Acide 6-méthoxy-1,2,3,4-tétrahydro-(2S)-2-naphtoïque**

On suit la méthode décrite dans l'Exemple 1, à partir de 4 g (0,018 mole) d'ester de départ au lieu de 3 g, en arrêtant la réaction enzymatique lorsque 37 ml de NaOH 0,25 N ont été consommés, en additionnant du bicarbonate de sodium jusqu'à pH 8. Ensuite on extrait à l'éther éthylique, on sépare les deux phases, on sèche la phase organique sur du sulfate de sodium et on évapore le solvant sous pression réduite. On obtient 1,85 g de méthyl 6-méthoxy-1,2,3,4-tétrahydro-(2S)-2-naphtoate (e.e. 92 %-HPLC effectuée dans les conditions (e)). On traite l'isomère (*S*) de l'ester ainsi obtenu avec une solution de NaOH, on acidifie à l'aide d'acide chlorhydrique 1N et on obtient 1,6 g de l'acide indiqué en titre qu'on cristallise dans de l'acétate d'éthyle. P.f. 129-130°C; $[\alpha]_D^{20}$ = - 57,3° (c =1,4 %, $CHCl_3$); e.e. 93,0 % (HPLC effectuée dans les conditions (a)).

**EXEMPLE 4**

**Acide 7-méthoxy-1,2,3,4-tétrahydro-(2R)-2-naphtoïque.**

On dissout S g (0,022 mole) de méthyl 7-méthoxy-1,2,3,4-tétrahydro-2-naphtoate dans 175 ml de *tert*-butanol et on ajoute à la solution ainsi préparée 500 ml de tampon phosphate pH = 7. Le pH de la solution, qui augmente jusqu'à une valeur de 7,3 - 7,5, est ramené à 7,1 par addition de HCl 1N. On ajoute au mélange 3,5 g d'enzyme PPL Sigma (indiquée comme "porcine pancreatic lipase" type II, crude, Sigma L-3126 - 39 U/mg using triacetine). Au fur et à mesure que la réaction s'effectue, le pH tend à baisser mais on le maintient constant par addition d'une solution 0,25 N de NaOH. Après 6 heures, lorsque environ 31,75 ml de NaOH 0,25 N ont été consommés, on ajoute à la solution du bicarbonate de sodium jusqu'à pH 8 environ, pour arrêter la réaction par inactivation de l'enzyme. On extrait dans de l'éther éthylique et on sépare les deux phases. La phase organique contient l'ester, principalement de configuration (*S*) qui n'a pas réagi et qui peut être récupéré comme indiqué dans l'Exemple 5. On acidifie la phase aqueuse à l'aide d'acide sulfurique concentré et on filtre le précipité formé. On obtient ainsi 1,25 g de l'acide indiqué en titre. $[\alpha]_D^{20}$ = + 43,67° (c =1,4 %, $CHCl_3$); e.e. 93,27 % (HPLC effectuée dans les conditions (a)). Ce produit est égal à celui décrit dans la Préparation (G) (i) de EP-A-436435, obtenu après 10 cristallisations.
Deux autres préparations effectuées dans les mêmes conditions ont donné le même produit ayant un e.e. de 91,65% et 93,52% (HPLC effectuée dans les conditions (a)).

**EXEMPLE 5**

**Acide 7-méthoxy-1,2,3,4-tétrahydro-(2S)-2-naphtoïque**

La phase organique résiduelle de l'Exemple 4 est séchée sur du sulfate de sodium et évaporée sous pression réduite. On obtient 3,39 g de l'ester contenant principalement l'isomère (*S*) (e.e. 37,6 % - HPLC effectuée dans les conditions (b)) qu'on distille (Eb = 130-135°C à 0,1 mbar). On dissout 3 g (0,014 mole) de l'ester ainsi récupéré dans 100 ml de *tert*-butanol; on ajoute à la solution ainsi obtenue 300 ml de tampon phosphate à pH 7. On porte à pH 7,04 par addition d'acide sulfurique à 5% et on ajoute 2 g de PPL Sigma (indiquée comme "porcine pancreatic lipase" type II, crude, Sigma L-3126 - 39 U/mg using triacetine). Au fur et à mesure que la réaction s'effectue, le pH baisse; on le maintient constant par addition d'une solution 0,25 N de NaOH. Après 6 heures environ, lorsque 15ml de NaOH 0,25 N ont été consommés, on ajoute du bicarbonate de sodium jusqu'à pH = 8 et on extrait à l'éther éthylique puis on sépare les deux phases, on élimine la phase aqueuse contenant l'acide 7-méthoxy-1,2,3,4-tétrahydro-(2R)-2-naphtoïque qui peut être récupéré, on sèche la phase organique sur du sulfate de sodium et on évapore le solvant sous pression réduite. On obtient 1,5 g de méthyl 7-méthoxy-1,2,3,4-tétrahydro-(2S)-2-naphtoate (e.e. 92,3% - HPLC effectuée dans les conditions (b)). On traite l'isomère (*S*) de l'ester ainsi obtenu avec une solution de NaOH, on acidifie à l'aide d'acide chlorhydrique 1N et on obtient 1,1 g de l'acide indiqué en titre. e.e. 92,3%. Ce produit est égal à celui décrit dans EP-A-436 435, obtenu après 10 cristallisations.
Deux autres préparations effectuées selon les mêmes conditions ont donné le produit du titre ayant les caractéristiques suivantes:
e.e. 91,6 %; $[\alpha]_D^{20}$ = - 44,6° (c =1,4 %, $CHCl_3$);
e.e. 93 %; $[\alpha]_D^{20}$ = - 44,3° (c =1,4 %, $CHCl_3$);

**EXEMPLE 6**

**Chlorhydrate de 7-méthoxy-1,2,3,4-tétrahydro-(2R)-2-naphtaleneamine**

A une solution de 2,5 g (0,0120 mole) d'acide 7-méthoxy-1,2,3,4-tétrahydro-(2R)-2-naphtoïque dans 30 ml d'acétone on ajoute lentement à la température de -10°C à -5°C, une solution de 2 ml (0,0140 mole) de triéthylamine dans 20 ml d'acétone et ensuite 1,6 ml (0,0161 mole) de chloroformiate d'éthyle dans 20 ml d'acétone. On agite à -5° C pendant 2 heures puis on ajoute, goutte à goutte, une solution de 1,3 g (0,0193 mole) de $NaN_3$ dans 10 ml d'eau. Après 1 heure à -5° C, on verse le mélange dans 200 ml d'eau et on extrait au toluène. On sépare les deux phases, on sèche la phase organique sur sulfate de sodium, on filtre et on chauffe la solution à 100°C pendant 1,5 heures. On évapore le solvant sous pression réduite, on reprend le résidu dans 22 ml d'eau et 26 ml d'acide chlorhydrique à 37% et on chauffe au reflux pendant 3,5 heures. Après refroidissement, on porte la solution à pH basique par addition de NaOH 3,5 N, on extrait à l'éther éthylique, on sèche la phase organique sur sulfate de sodium et on évapore le solvant sous pression réduite. On prépare le chlorhydrate de la base ainsi obtenue à l'aide d'éther éthylique saturé en acide chlorhydrique. On obtient 1,6 g du composé indiqué en titre qu'on cristallise dans de l'isopropanol. On obtient 1,2 g de produit. P.f. 205°C-207°C;$[\alpha]_D^{20}$ = + 66,6° (c = 0,5% MeOH).

**EXEMPLE 7**

**Chlorhydrate de 7-méthoxy-1,2,3,4-tétrahydro-(2S)-2-naphtaleneamine.**

On suit la méthode de l'Exemple 6 en utilisant 2,5 g d'acide 7-méthoxy-1,2,3,4-tétrahydro-(2S)-2-naphtoïque en tant que produit de départ. On obtient 1 g du composé indiqué en titre P.f. 205° C - 207° C; $[\alpha]_D^{20}$ = - 66,4°(c = 0,4% MeOH).

**EXEMPLE 8**

**Chlorhydrate de (2R)-2-aminométhyl-6-méthoxy-1,2,3,4-tétrahydronaphtalène**

(i) 6-méthoxy-1,2,3,4-tétrahydronaphtalene-(2R)-2-carboxamide.

A une solution de 7,01 g (0,034 mole) d'acide 6-méthoxy-1,2,3,4-tétrahydro-(2R)-2-naphtoïque dans 150 ml de chlorure de méthylène, en conditions anhydres, on ajoute goutte à goutte 3,48 ml (0,038 mole) de chlorure d'oxalyle et quelques gouttes de diméthylformamide. On laisse agiter à la température ambiante pendant 5 heures puis on ajoute ce mélange, goutte à goutte, à une solution de 21,52 ml (0,102 mole) d'hexaméthyldisilazane dans 15 ml de chlorure de méthylène anhydre. On laisse agiter le mélange à la température ambiante pendant une nuit. On ajoute 25 ml de méthanol et on agite pendant 30 minutes. On verse dans 200 ml d'acide sulfurique à 5% et on extrait au chlorure de méthylène. On sépare les deux phases, on lave la phase organique avec une solution saturée de chlorure d'ammonium, on sèche sur sulfate de sodium et on évapore le solvant sous pression réduite.

On obtient 11,8 g de produit que l'on purifie par chromatographie flash en éluant avec un mélange chlorure de méthylène/méthanol = 9/1. On obtient 4,64 g de 6-méthoxy-1,2,3,4-tétrahydronaphtalene-(2R)-2-carboxamide. e. e. 94,5% (HPLC effectuée dans les conditions (c)); $[\alpha]_D^{20}$ = +52,9° (c = 1,4% $CHCl_3$).

(ii) Chlorhydrate de (2R)-2-aminométhyl-6-méthoxy-1,2,3,4-tétrahydronaphtalène

On soumet le composé obtenu dans l'étape (i) ci-dessus à la réaction décrite dans EP-A-436435, Préparation (O) (iii). On obtient le chlorhydrate de (2R)-2-aminométhyl-6-méthoxy-1,2,3,4-tétrahydronaphtalène. P.f. 255-256°C; $[\alpha]_D^{20}$ = +75,3° (c = 1,4% $CHCl_3$).

## EXEMPLE 9

**Acide 8-méthoxy-1,2,3,4-tétrahydro-(2R)-2-naphtoïque.**

On dissout 0,743 g (0,0034 mole) de méthyl 8-méthoxy-1,2,3,4-tétrahydro-2-naphtoate dans 26 ml de *tert*-butanol et on ajoute à la solution ainsi préparée 75 ml de tampon phosphate à pH 7. La valeur du pH de la solution, qui augmente jusqu'à 7,3 - 7,5, est ramenée à 7,1 par addition de HCl 1N. On ajoute au mélange 0,836 g d'enzyme PPL Sigma (indiquée comme "porcine pancreatic lipase" type II, crude, Sigma L-3126 - 46 U/mg using triacetine). Au fur et à mesure que la réaction s'effectue, le pH tend à baisser mais on le maintient constant par addition d'une solution 0,25 N de NaOH en contrôlant le pH par un appareil automatique de titration. Après 6,5 heures, on verse le mélange dans une solution de bicarbonate de sodium à 5% pour arrêter la réaction par inactivation de l'enzyme. On extrait dans de l'éther éthylique et on sépare les deux phases. La phase organique contient l'ester, principalement de configuration (*S*) qui n'a pas réagi et qui peut être récupéré comme indiqué dans l'Exemple 10 ci-dessous. On acidifie la phase aqueuse à l'aide d'acide sulfurique à 10% et on extrait au chloroforme; on filtre la phase organique sur Célite®, on sèche sur du sulfate de sodium et on évapore le solvant sous préssion réduite. On obtient 0,303 g de l'acide indiqué en titre qu'on cristallise dans l'éther isopropylique. e.e. 95,2 % (HPLC effectuée dans les conditions (d)); P.f. 137-138°C; $[\alpha]_D^{25}$ = +54,6° (c = 1,4%, $CH_3OH$).

## EXEMPLE 10

**Acide 8-méthoxy-1,2,3,4-tétrahydro-(2S)-2-naphtoïque.**

La phase organique residuelle de l'Exemple 9 est séchée sur du sulfate de sodium et évaporée sous pression réduite. On obtient 0,403 g de méthyl 8-méthoxy-1,2,3,4-tétrahydro-(**2S**)-**2**-naphtoate (e.e. 90,4 % - HPLC effectuée dans les conditions (d)). On traite l'isomère (S) de l'ester ainsi directement obtenu avec une solution de 10 ml de méthanol et 4 ml de NaOH 1N. On chauffe au reflux pendant 2 heures, on concentre la solution, on reprend le résidu avec une solution aqueuse saturée de NaCl et on lave à l'éther éthylique. On acidifie la phase aqueuse jusqu'à pH 1 par addition d'acide sulfurique à 5%, on extrait au chlorure de méthylène, on sèche sur du sulfate de sodium et on évapore le solvant sous pression réduite. On obtient 0,39 g du produit du titre qu'on purifie par chromatographie sur colonne de gel de silice en éluant avec un mélange $CH_2Cl_2$/MeOH = 95/5. Le produit est ultérieurement purifié par cristallisation dans 10 ml d'éthanol. P.f. 140,5°C. $[\alpha]_D^{25}$ = -50,5° (c = 1,4%, $CHCl_3$). e.e. = 90,0 % (HPLC effectuée dans les conditions (d)).

Une autre préparation effectuée dans les mêmes conditions a donné le produit du titre ayant un e.e. de 94,8 %.

La configuration absolue de l'acide du titre a été démontrée, comme décrit dans l'exemple 13 ci-après, par transformation du même acide en le 8-méthoxy-2-(N-benzyl)amino-1,2,3,4-tétrahydronaphtalène, dont la configuration absolue (S) est connue.

## EXEMPLE 11

**Acide 5-méthoxy-1,2,3,4-tétrahydro-(2R)-2-naphtoïque.**

On dissout 0,8 g (0,0036 mole) de méthyl 5-méthoxy-1,2,3,4-tétrahydro-2-naphtoate dans 28 ml de *tert*-butanol et on ajoute à la solution ainsi préparée 80 ml de tampon phosphate à pH 7. La valeur du pH de la solution, qui augmente jusqu'à 7,3 - 7,5, est ramenée à 7,1 par addition de $H_2SO_4$ dilué. On ajoute au mélange 0,450 g d'enzyme PPL Sigma (indiquée comme "porcine pancreatic lipase" type II, crude, Sigma L-3126 - 46 U/mg using triacetine). Au fur et à mesure que la réaction s'effectue, le pH tend à baisser mais on le maintient constant par addition d'une solution 0,25 N de NaOH en contrôlant le pH par un appareil automatique de titration. Après 8 heures, on ajoute à la solution du bicarbonate de sodium jusqu'à obtenir pH 7,7, pour arrêter la réaction par inactivation de l'enzyme. On extrait dans de l'éther isopropylique et on sépare les deux phases. La phase organique contient l'ester, principalement de configuration (*S*) qui n'a pas réagi. On acidifie la phase aqueuse à l'aide d'acide sulfurique concentré et on extrait à l'éther éthylique, on sèche la phase organique sur du sulfate de sodium et on évapore le solvant sous préssion réduite. On cristallise le produit obtenu dans de l'éther isopropylique. On obtient 0,12 g du composé du titre. P.f. 150-152°C; $[\alpha]_D^{20}$ = +55,9° (c = 1,4%, $CHCl_3$) ; e.e. 85,6 % (HPLC effectuée dans les conditions (e)).

Une autre préparation effectuée selon la même procédure a donné le produit du titre ayant un e.e. de 83%.

**EXEMPLE 12**

**Acide 5-méthoxy-1,2,3,4-tétrahydro-(2S)-2-naphtoïque.**

On suit la méthode décrite dans l'Exemple 11, à partir de 0,74 g d'ester racémique de départ au lieu de 0,8 g, 26 ml *tert*-butanol, 75 ml de tampon phosphate et 0,83 g d'enzyme PPL Sigma (indiquée comme "porcine pancreatic lipase" type II, crude, Sigma L-3126- 36 U/mg using triacétine). On arrête la réaction lorsque 8 ml de NaOH 0,25 N sont consommés en additionnant du bicarbonate de sodium jusqu'à pH 8. On extrait à l'éther éthylique, on sépare les deux phases, on sèche la phase organique sur du sulfate de sodium et on évapore le solvant sous préssion réduite. On obtient 0,37 g de méthyl 5-méthoxy-1,2,3,4-tétrahydro-(2S)-2-naphtoate ayant un e.e. de 60%. On traite ce produit avec 3 ml de méthanol et 3 ml d'une solution aqueuse de NaOH 2 N et on agite le mélange à la température ambiante pendant 4 heures. On dilue à l'eau et on lave à l'éther éthylique. On acidifie la phase aqueuse avec de l'acide chlorhydrique concentré et on extrait à l'acétate d'éthyle. On lave la phase organique à l'eau, on sèche sur du sulfate de sodium et on évapore le solvant sous pression réduite. On obtient le composé du titre qu'on cristallise dans 5 ml d'éther isopropylique. P.f. 150-152°C; $[\alpha]_D^{20}$ = -49,6° (c = 1,4%, $CHCl_3$) ; e.e 58,9 % (HPLC effectuée dans les conditions (e)).

**EXEMPLE 13**

**Chlorhydrate de 8-méthoxy-(2R)-2-(N-benzyl)amino-1,2,3,4-tétrahydronaphtalène**

(i) 8-méthoxy-(2R)-2-(N-*tert*-butoxycarbonyl)amino-1,2,3,4-tétrahydronaphtalène.

A un mélange de 0,3 g (1,5 mmole) d'acide 8-méthoxy-1,2,3,4-tétrahydro-(2R)-2-napthoïque dans 7 ml d'alcool *tert*-butylique sous azote, on ajoute 0,23 ml (1,65 mmole) de triéthylamine et 0,35 ml (1,65 mmole) de diphénylphosphorylazide (DPPA). On chauffe le mélange au reflux pendant 20 heures, on verse dans 20 ml d'une solution aqueuse de bicarbonate de sodium à 5%, on extrait au chloroforme, on lave la phase organique avec une solution d'acide phosphorique à 3% puis avec une solution de NaOH 1N. On sèche la phase organique sur du sulfate de sodium et on évapore le solvant sous pression réduite. On obtient 0,38 g d'un solide blanc qui est directement soumis à la réaction de l'étape (ii).

(ii) 8-méthoxy-(2R)-2-(N-benzyl-N-*tert*-butoxycarbonyl)amino-1,2,3,4-tétrahydronaphtalène.

A une suspension de 0,021 g (0,72 mmole) de NaH à 80% dans 3 ml de tétrahydrofurane, sous azote, on ajoute 0,2 g (0,72 mmole) du produit obtenu à l'étape (i) dans 1,5 ml de tétrahydrofurane. On chauffe le mélange à 60°C pendant 10 minutes, on y ajoute 0,086 g (0,72 mmole) de bromure de benzyle et on agite le mélange à 60°C pendant 8 heures. On verse le mélange dans 20 ml d'une solution aqueuse de bicarbonate de sodium à 5%, on extrait à l'éther éthylique, on sèche la phase organique sur du sulfate de sodium et on évapore le solvant sous pression réduite. On purifie le résidu par chromatographie sur colonne de gel de silice en éluant avec un mélange acétate d'éthyle/hexane = 1/4. On obtient 0,1 g du composé du titre qui est soumis à la réaction de l'étape (iii).

(iii) Chlorhydrate de 8-méthoxy-(2R)-2-(N-benzyl)amino-1,2,3,4-tétrahydronaphtalène.

On dissout 0,1 g du composé obtenu de l'étape précédente dans 3 ml d'acide trifluoroacétique et on agite à la température ambiante pendant 12 heures. On évapore le solvant, on reprend le résidu dans du méthanol et on évapore à sec le solvant. On répète cette opération 3 fois. On traite l'huile obtenue avec du méthanol saturé en acide chlorhydrique et on évapore le solvant sous pression réduite. On obtient le composé du titre qu'on cristallise dans un mélange méthanol/éther isopropylique. P.f. 237-238°C; $[\alpha]_D^{25}$ = +59,2° (c = 1%, $CH_3OH$); e.e. 93% (Référence Acta Chem. Scand. B, 1988, 42 : 231).

**EXEMPLE 14**

**Chlorhydrate de 8-méthoxy-(2R)-2-amino-1,2,3,4-tétrahydronaphtalène**

(i) Acide 8-méthoxy-1,2,3,4-tétrahydro-(2R)-2-naphtoïque.

On dissout 1,1 g (0,0051 mole) de méthyl 8-méthoxy-1,2,3,4-tétrahydro-2-naphtoate dans 39 ml de *tert*-butanol et on ajoute à la solution ainsi préparée 112 ml de tampon phosphate à pH 7. La valeur du pH de la solution, qui augmente jusqu'à 7,3 - 7,5, est ramenée à 7,1 par addition de HCl 1N. On ajoute au mélange 1,25 g d'enzyme PPL Sigma (indiquée comme "porcine pancreatic lipase" type II, crude, Sigma L-3126 - 46 U/mg using triacetine). Au fur et à mesure que la réaction s'effectue, le pH tend à baisser mais on le maintient constant par addition d'une solution 0,25 N de NaOH en contrôlant le pH par un appareil automatique de titration. Après 7 heures, on verse le mélange dans une solution de bicarbonate de sodium à 5% pour arrêter la réaction par inactivation de l'enzyme. On extrait dans de l'éther éthylique et on sépare les deux phases. La phase organique contient l'ester, principalement de configuration (*S*) qui n'a pas réagi et qui peut être récupéré. On acidifie la phase aqueuse à l'aide d'acide sulfurique à 10% et on extrait au chloroforme; on filtre la phase organique sur Célite®, on sèche sur du sulfate de sodium et on évapore le solvant sous pression réduite. On obtient 0,42 g de l'acide indiqué en titre. e.e. 98 % (HPLC effectuée dans les conditions (d)).

(ii) 8-méthoxy-(2R)-2-(N-*tert*-butoxycarbonyl)amino-1,2,3,4-tétrahydronaphtalène.

A un mélange de 0,4 g (2 mmole) d'acide obtenu à l'étape précédente dans 9,4 ml d'alcool *tert*-butylique sous azote, on ajoute 0,3 ml (2,2 mmole) de triéthylamine et 0,46 ml (2,2 mmole) de diphénylphosphorylazide (DPPA). On chauffe le mélange au reflux pendant 20 heures, on verse dans 26 ml d'une solution aqueuse de bicarbonate de sodium à 5%, on extrait au chloroforme, on lave la phase organique avec une solution d'acide phosphorique à 3% puis avec une solution de NaOH 1N. On sèche la phase organique sur du sulfate de sodium et on évapore le solvant sous pression réduite. On obtient 0,52 g d'un solide blanc qui est directement soumis à la réaction de l'étape (iii) suivante.

(iii) Chlorhydrate de 8-méthoxy-(2R)-2-amino-1,2,3,4-tétrahydronaphtalène.

On agite à la température ambiante pendant 12 heures un mélange de 0,17 g (0,61 mmole) du produit obtenu à l'étape précédente dans 7 ml d'acide trifluoroacétique. On évapore le solvant sous pression réduite, on reprend le résidu dans du méthanol et on évapore le solvant sous pression réduite. On répète cette opération 3 fois. On traite le résidu avec du méthanol saturé en acide chlorhydrique et on évapore le solvant. On cristallise le résidu dans un mélange méthanol/éther éthylique. On obtient le composé du titre. P.f. 244°C déc.; $[\alpha]_D^{25}$ = +51,2° (c = 1%, $CH_3OH$); e.e.= 94%.

## EXEMPLE 15

**Chlorhydrate de 8-méthoxy-(2S)-2-amino-1,2,3,4-tétrahydronaphtalène**

En suivant la procédure décrite dans l'Exemple 14 mais en utilisant 0,26 g d'acide 8-méthoxy-1,2,3,4-tétrahydro-(2S)-2-naphtoïque en tant que produit de départ, on obtient 0,3 g du composé du titre. P.f. 242-244°C déc.; $[\alpha]_D^{25}$ = -48,5° (c = 1%, $CH_3OH$); e.e. = 89,2%.

## EXEMPLE 16

**Chlorhydrate de (2R)-2-aminométhyl-6-méthoxy-1,2,3,4-tétrahydronaphtalène**

(i) Acide 6-méthoxy-1,2,3,4-tétrahydro-(2R)-2-naphtoïque.

On dissout 5 g de méthyl 6-méthoxy-1,2,3,4-tétrahydro-2-naphtoate dans 170 ml de *tert*-butanol et on ajoute à la solution ainsi préparée 500 ml de tampon phosphate à pH 7. La valeur du pH de la solution, qui augmente jusqu'à 7,3 - 7,5, est ramenée à 7,1 par addition de HCl 1N. On ajoute au mélange 2,5 g d'enzyme PPL Sigma (indiquée comme "porcine pancreatic lipase" type II, crude, Sigma L-3126 - 50 U/mg using triacetine). Au fur et à mesure que la réaction s'effectue, le pH tend à baisser mais on le maintient constant par addition d'une solution 0,25 N de NaOH en contrôlant le pH par un appareil automatique de titration. Après 5 heures on ajoute à la solution du bicarbonate de sodium jusqu'à pH 8 environ, pour arrêter la réaction par inactivation de l'enzyme. On extrait dans de l'éther éthylique et on sépare les deux phases. La phase organique contient l'ester, principalement de configuration (*S*) qui n'a pas réagi et qui peut être récupéré. On acidifie la phase aqueuse à l'aide d'acide sulfurique concentré et on filtre le précipité formé. On obtient ainsi 1,8 g de l'acide indiqué en titre qu'on cristallise dans l'acétate d'éthyle. e.e. 93%.

(ii) 6-méthoxy-1,2,3,4-tétrahydronaphtalene-(2R)-2-carboxamide.

A une solution de 1,8 g (8,7 mmole) d'acide 6-méthoxy-1,2,3,4-tétrahydro-(2R)-2-naphtoïque dans 39 ml de chlorure de méthylène, en conditions anhydres, on ajoute goutte à goutte 0,9 ml (9,7 mmole) de chlorure d'oxalyle et quelques gouttes de diméthylformamide. On laisse agiter à la température ambiante pendant 5 heures puis on ajoute ce mélange, goutte à goutte, à une solution de 5,5 ml (26 mmole) d'hexaméthyldisilazane dans 4 ml de chlorure de méthylène anhydre. On laisse agiter le mélange à la température ambiante pendant une nuit. On ajoute 6 ml de méthanol et on agite pendant 30 minutes. On verse dans 50 ml d'acide sulfurique à 5% et on extrait au chlorure de méthylène. On sépare les deux phases, on lave la phase organique avec une solution saturée de chlorure d'ammonium, on sèche sur sulfate de sodium et on évapore le solvant sous pression réduite. On purifie le produit brut par chromatographie flash en éluant avec un mélange chlorure de méthylène/méthanol = 9/1. On obtient 1,5 g de 6-méthoxy-1,2,3,4-tétrahydronaphtalene-(2R)-2-carboxamide. e.e. 95%.

(iii) Chlorhydrate de (2R)-2-aminométhyl-6-méthoxy-1,2,3,4-tétrahydronaphtalène.

On soumet le composé obtenu dans l'étape (ii) ci-dessus à la réaction décrite dans EP-A-436435, Préparation (O) (iii). On obtient le chlorhydrate de (2R)-2-aminométhyl-6-méthoxy-1,2,3,4-tétrahydronaphtalène. P.f. 255-256°C; $[\alpha]_D^{20}$ = +75,3° (c = 1,4% $CHCl_3$).

## Revendications

1. Procédé pour la préparation des composés de formule (I) sous forme optiquement active

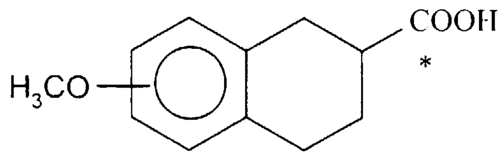

(I)

caractérisé en ce que:

(a) on soumet un ester racémique de formule

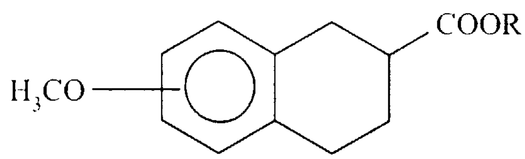

(II)

dans laquelle R est un alkyle en $C_1$-$C_3$, à une hydrolyse par la lipase de pancréas de porc ; puis
(b) lorsque environ 50 % de l'ester est hydrolysé en acide, on interrompt l'hydrolyse par inactivation de l'enzyme et on récupère l'ester de configuration (*S*) de formule

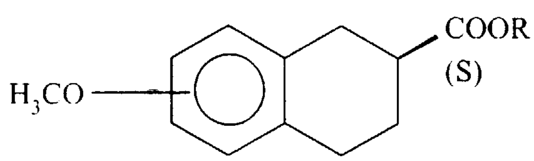

(II')

qui n'a pas réagi, enfin
(c) soit on hydrolyse ledit ester de formule (II') et on isole l'acide de configuration (*S*) de formule

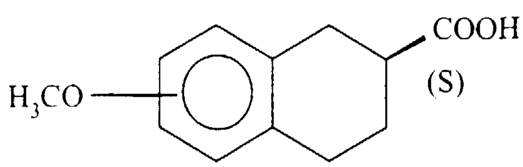

(I')

soit on isole l'acide de configuration (*R*) de formule

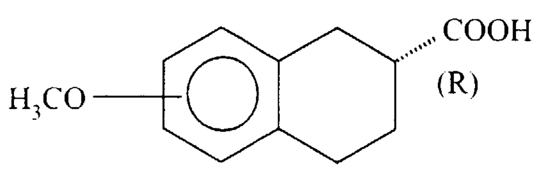

(I")

tel qu'obtenu de la réaction d'hydrolyse lipasique après interruption de l'hydrolyse à l'étape (b).

2. Procédé selon la revendication 1, caractérisé en ce que dans l'étape (b) on interrompt l'hydrolyse enzymatique juste avant que 50% du composé (II) soit hydrolysé, par addition d'hydroxyde de sodium jusqu'à pH basique, on

extrait le composé (II') qui n'a pas réagi dans un solvant organique immiscible à l'eau puis on élimine la phase organique et, dans l'étape (c) on traite la phase aqueuse avec un acide minéral ou organique et on sépare l'acide de configuration (*R*) de formule (I") ainsi précipité.

**3.** Procédé selon la revendication 1, caractérisé en ce que dans l'étape (b) on interrompt l'hydrolyse enzymatique juste après que 50% du composé (II) est hydrolysé, par addition d'hydroxyde de sodium jusqu'à pH basique, on extrait le composé (II') qui n'a pas réagi dans un solvant organique immiscible à l'eau puis on élimine la phase aqueuse, on isole l'ester (II') et, dans l'étape (c) on hydrolyse ledit ester de formule (II') et on isole l'acide de configuration (*S*) de formule (I').

**4.** Procédé selon l'une quelconque des revendications 1 à 3, caractérisé en ce que l'étape (a) est effectuée en milieu aqueux.

**5.** Procédé selon la revendication 4, caractérisé en ce que l'étape (a) est effectuée en milieu aqueux en présence d'un tampon à pH 7.

**6.** Procédé selon l'une quelconque des revendications 1 à 5, caractérisé en ce que la température de réaction de l'étape (a) est comprise entre 0°C et +60°C.

**7.** Procédé selon l'une quelconque des revendications 1 à 6, caractérisé en ce que comme produit de départ, on utilise un composé de formule (II) dans laquelle R est un méthyle.

**8.** Procédé pour la préparation de composés optiquement actifs de formule (III)

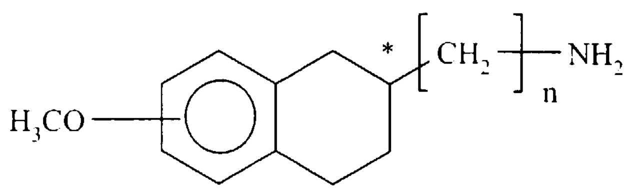

(III)

dans laquelle n est 0 ou 1 et l'astérique (*) désigne l'atome de carbone chiral dans sa forme (R) ou (S), et de ses sels, caractérisé en ce que

(a) on soumet un ester racémique de formule

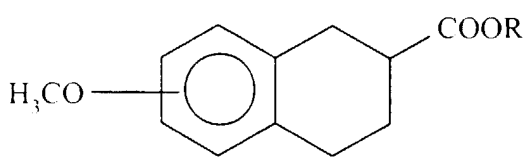

(II)

dans laquelle R est un alkyle en $C_1$-$C_3$, à une hydrolyse par la lipase de pancréas de porc ; puis
(b) lorsque environ 50 % de l'ester est hydrolysé en acide, on interrompt l'hydrolyse par inactivation de l'enzyme et on récupère l'ester de configuration (*S*) de formule

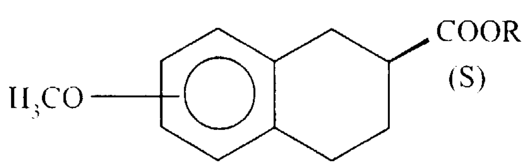

(II')

qui n'a pas réagi, puis
(c) <u>soit</u> on hydrolyse ledit ester de formule (II') et on isole l'acide de configuration (*S*) de formule

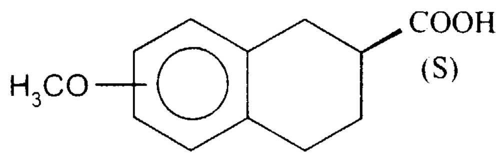

(I')

soit on isole l'acide de configuration (*R*) de formule

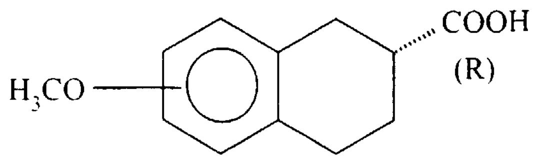

(I")

tel qu'obtenu de la réaction d'hydrolyse lipasique, après interruption de l'hydrolyse à l'étape (b), enfin
(d) soit on soumet les acides isomères à la réaction avec un azoture selon la réaction de Curtius pour isoler une amine de formule (III) où n est 0,
soit on transforme les acides isomères (I') et (I") ou l'ester (II') en l'amide correspondant et on réduit ce dernier pour isoler une amine de formule (III) où n est 1;
lesdites amines étant isolées sous forme de base libre ou d'un de leur sels d'addition d'acide, ou transformées en un de leurs sels d'addition d'acides.

**9.** Composé de formule

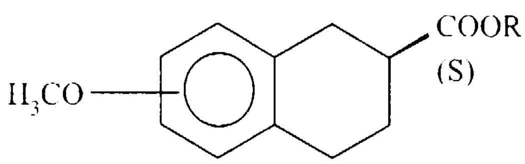

(II')

dans laquelle R est un alkyle en $C_1$-$C_3$.

**10.** Composé selon la revendication 9, choisi parmi le 6-méthoxy-1,2,3,4-tétrahydro-(2S)-2-naphtoate de méthyle, le 7-méthoxy-1,2,3,4-tétrahydro-(2S)-2-naphtoate de méthyle, le 8-méthoxy-1,2,3,4-tétrahydro-(2S)-2-naphtoate de méthyle et le 5-méthoxy-1,2,3,4-tétrahydro-(2S)-2-naphtoate de méthyle.

**11.** Acide 8-méthoxy-1,2,3,4-tétrahydro-(2R)-2-napthoïque.

**12.** Acide 8-méthoxy-1,2,3,4-tétrahydro-(2S)-2-napthoïque.

**Patentansprüche**

**1.** Verfahren zur Herstellung von Verbindungen der Formel (I) in optisch aktiver Form

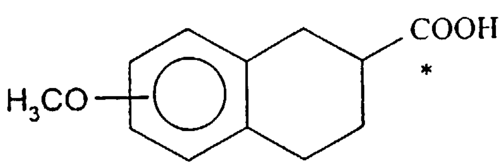

(I),

dadurch gekennzeichnet, daß:

(a) ein racemischer Ester der Formel

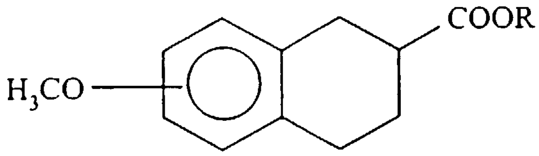

(II),

worin R ein $C_1$-$C_3$-Alkyl ist, einer Hydrolyse durch Schweine-Pankreaslipase unterworfen wird; dann

(b) wenn ungefähr 50 % des Esters in Säure hydrolysiert sind, die Hydrolyse durch die Inaktivierung des Enzyms unterbrochen wird, und der Ester mit (S)-Konfiguration der Formel

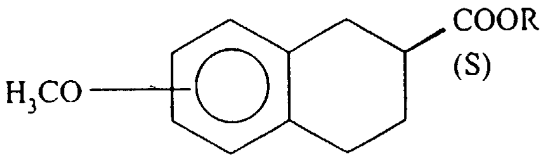

(II'),

der nicht reagiert hat, gewonnen wird, schließlich

(c) entweder der Ester der Formel (II') hydrolysiert wird, und die Säure mit (S)-Konfiguration der Formel

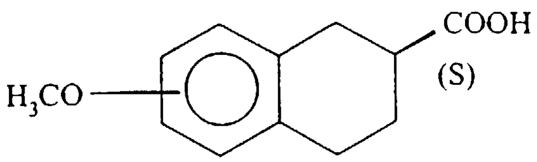

(I')

isoliert wird,

oder die Säure mit (R)-Konfiguration der Formel

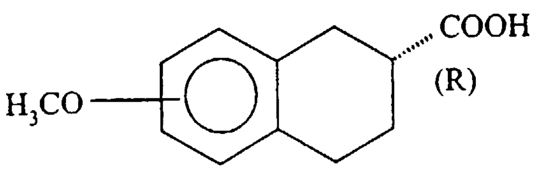

(I"),

wie aus der Lipase-Hydrolysereaktion nach Unterbrechung der Hydrolyse in Schritt (b) erhalten, isoliert wird.

2. Verfahren nach Anspruch 1, dadurch gekennzeichnet, daß in Schritt (b) die enzymatische Hydrolyse unterbrochen wird, gerade bevor 50 % der Verbindung (II) hydrolysiert sind, durch den Zusatz von Natriumhydroxid bis auf einen basischen pH, die Verbindung (II'), die nicht reagiert hat, in einem mit Wasser nicht mischbaren organischen Lösungsmittel extrahiert wird, dann die organische Phase eliminiert wird, und in Schritt (c) die wässerige Phase mit einer Mineral- oder organischen Säure behandelt wird, und die so ausgefällte Säure mit (R)-Konfiguration der Formel (I") abgetrennt wird.

3. Verfahren nach Anspruch 1, dadurch gekennzeichnet, daß in Schritt (b) die enzymatische Hydrolyse unterbrochen wird, gerade nachdem 50 % der Verbindung (II) hydrolysiert sind, durch den Zusatz von Natriumhydroxid bis auf einen basischen pH, die Verbindung (II'), die nicht reagiert hat, in einem mit Wasser nicht mischbaren organischen Lösungsmittel extrahiert wird, dann die wässerige Phase eliminiert wird, der Ester (II') isoliert wird, und in Schritt (c) der Ester der Formel (II') hydrolysiert wird, und die Säure mit (S)-Konfiguration der Formel (I') isoliert wird.

**4.** Verfahren nach einem der Ansprüche 1 bis 3, dadurch gekennzeichnet, daß der Schritt (a) in wässerigem Medium durchgeführt wird.

**5.** Verfahren nach Anspruch 4, dadurch gekennzeichnet, daß der Schritt (a) in wässerigem Medium in Anwesenheit eines Puffers bei pH 7 durchgeführt wird.

**6.** Verfahren nach einem der Ansprüche 1 bis 5, dadurch gekennzeichnet, daß die Reaktionstemperatur von Schritt (a) zwischen 0°C und +60°C liegt.

**7.** Verfahren nach einem der Ansprüche 1 bis 6, dadurch gekennzeichnet, daß als Ausgangsprodukt eine Verbindung der Formel (II), worin R ein Methyl bedeutet, verwendet wird.

**8.** Verfahren zur Herstellung optisch aktiver Verbindungen der Formel (III)

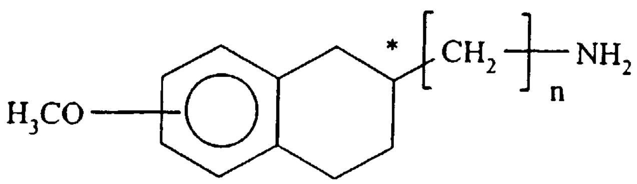

(III),

worin n Null oder 1 ist, und das Sternchen (*) ein chirales Kohlenstoffatom in seiner (R)- oder (S)-Form bedeutet, und ihrer Salze, dadurch gekennzeichnet, daß

(a) ein racemischer Ester der Formel

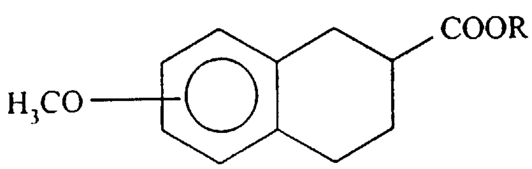

(II),

worin R ein $C_1$-$C_3$-Alkyl ist, einer Hydrolyse durch Schweine-Pankreaslipase unterworfen wird; dann
(b) wenn ungefähr 50 % des Esters in Säure hydrolysiert sind, die Hydrolyse durch die Inaktivierung des Enzyms unterbrochen wird, und der Ester mit (S)-Konfiguration der Formel

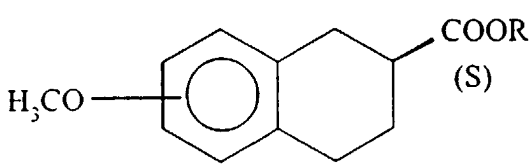

(II'),

der nicht reagiert hat, gewonnen wird, dann
(c) entweder der Ester der Formel (II') hydrolysiert wird, und die Säure mit (S)-Konfiguration der Formel

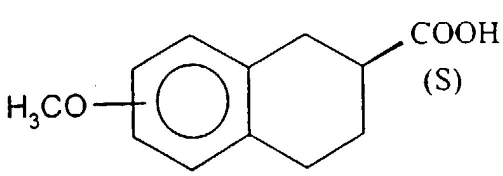

(I')

isoliert wird,
oder die Säure mit (R)-Konfiguration der Formel

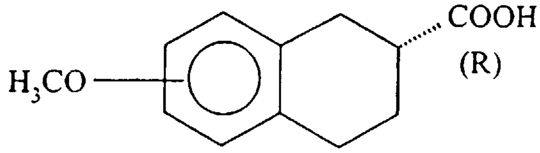

(I"),

wie aus der Lipase-Hydrolysereaktion nach Unterbrechung der Hydrolyse in Schritt (b) erhalten, isoliert wird, schließlich
(d) entweder die isomeren Säuren einer Reaktion mit einem Nitrid gemäß der Curtius-Reaktion unterworfen werden, um ein Amin der Formel (III), worin n 0 ist, zu isolieren,

oder die isomeren Säuren (I') und (I") oder der Ester (II') in ein entsprechendes Amid übergeführt werden, und dieses letztere reduziert wird, um ein Amin der Formel (III), worin n 1 ist, zu isolieren;
wobei die Amine in Form der freien Base oder eines ihrer Säureadditionssalze isoliert oder in eines ihrer Säureadditionssalze übergeführt werden.

**9.** Verbindung der Formel

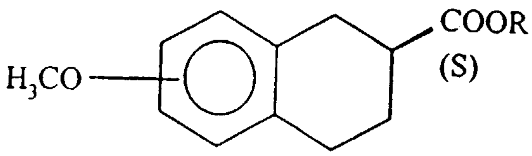

(II'),

worin R ein $C_1$-$C_3$-Alkyl ist.

**10.** Verbindung nach Anspruch 9, ausgewählt aus 6-Methoxy-1,2,3,4-tetrahydro-(2S)-2-methylnaphtoat, 7-Methoxy-1,2,3,4-tetrahydro-(2S)-2-methylnaphtoat, 8-Methoxy-1,2,3,4-tetrahydro-(2S)-2-methylnaphtoat und 5-Methoxy-1,2,3,4-tetrahydro-(2S)-2-methylnaphtoat.

**11.** 8-Methoxy-1,2,3,4-tetrahydro-(2R)-2-naphtoesäure.

**12.** 8-Methoxy-1,2,3,4-tetrahydro-(2S)-2-naphtoesäure.

**Claims**

**1.** A process for the preparation of the compounds of formula (I) in optically active form:

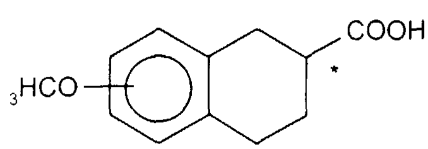

(I)

wherein:

(a) a racemic ester of the formula

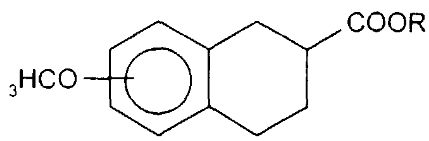

(II)

in which R is a ($C_1$-$C_3$)alkyl, is hydrolyzed with porcine pancreatic lipase; then

(b) when about 50% of the ester has been hydrolyzed to the acid, the hydrolysis is interrupted by inactivation of the enzyme and the unreacted ester of (S) configuration of the formula

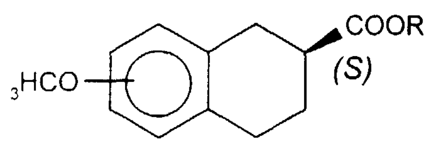

(II')

is recovered; and finally

(c) either said ester of formula (II') is hydrolyzed and the acid of (S) configuration of the formula

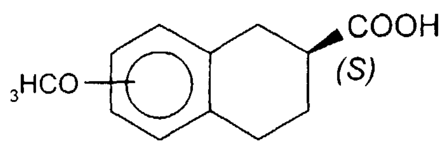

(I')

is isolated,

or the acid of (R) configuration of the formula

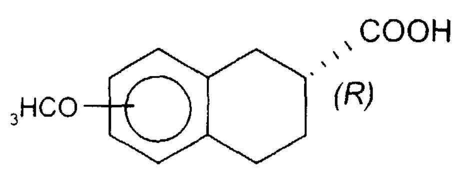

(I")

as obtained from the lipase hydrolysis reaction is isolated after interruption of the hydrolysis in step (b).

**2.** The process according to claim 1 wherein the enzymatic hydrolysis in step (b) is interrupted just before 50% of the compound (II) has been hydrolyzed, by the addition of sodium hydroxide until the pH is basic, the unreacted compound (II') is extracted into a water-immiscible organic solvent, the organic phase is then removed and, in step (c), the aqueous phase is treated with a mineral or organic acid and the acid of (R) configuration of formula (I") precipitated in this way is separated off.

**3.** The process according to claim 1 wherein the enzymatic hydrolysis in step (b) is interrupted just after 50% of the compound (II) has been hydrolyzed, by the addition of sodium hydroxide until the pH is basic, the unreacted compound (II') is extracted into a water-immiscible organic solvent, the aqueous phase is then removed, the ester (II') is isolated and, in step (c), said ester of formula (II') is hydrolyzed and the acid of (S) configuration of formula (I') is isolated.

**4.** The process according to any one of claims 1 to 3 wherein step (a) is carried out in an aqueous medium.

**5.** The process according to claim 4 wherein step (a) is carried out in an aqueous medium in the presence of a buffer at pH 7.

**6.** The process according to any one of claims 1 to 5 wherein the reaction temperature of step (a) is between 0°C and +60°C.

**7.** The process according to any one of claims 1 to 6 wherein the starting material used is a compound of formula (II) in which R is methyl.

**8.** A process for the preparation of optically active compounds of formula (III):

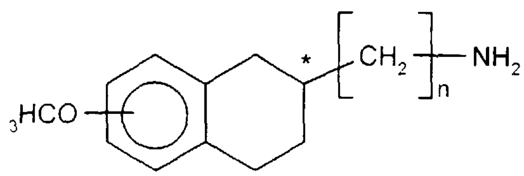

(III)

in which n is 0 or 1 and the asterisk (*) denotes the chiral carbon atom in its (R) or (S) form, and its salts, wherein

(a) a racemic ester of the formula

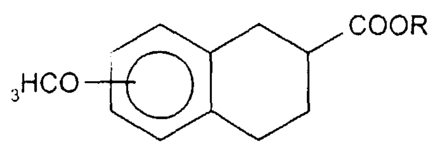

(II)

in which R is a ($C_1$-$C_3$)alkyl, is hydrolyzed with porcine pancreatic lipase; then
(b) when about 50% of the ester has been hydrolyzed to the acid, the hydrolysis is interrupted by inactivation of the enzyme and the unreacted ester of (S) configuration of the formula

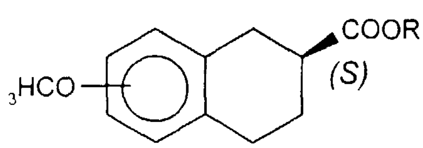

(II')

is recovered; then
(c) <u>either</u> said ester of formula (II') is hydrolyzed and the acid of (S) configuration of the formula

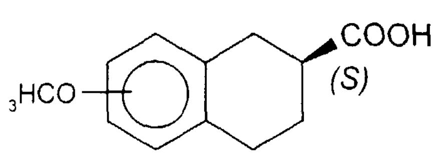

(I')

is isolated,
<u>or</u> the acid of (R) configuration of the formula

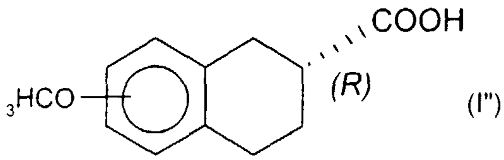

(I'')

as obtained from the lipase hydrolysis reaction is isolated after interruption of the hydrolysis in step (b); and finally

(d) either the isomeric acids are reacted with an azide by the Curtius reaction in order to isolate an amine of formula (III) in which n is 0,

or the isomeric acids (I') and (I'') or the ester (II') are converted to the corresponding amide and the latter is reduced in order to isolate an amine of formula (III) in which n is 1, said amines being isolated in the form of the free base or one of their acid addition salts, or converted to one of their acid addition salts.

**9.** A compound of the formula

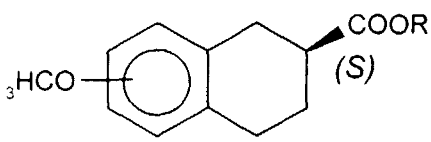

(II')

in which R is a $(C_1-C_3)$alkyl.

**10.** The compound according to claim 9, which is selected from methyl 6-methoxy-1,2,3,4-tetrahydro-(2S)-2-naphthoate, methyl 7-methoxy-1,2,3,4-tetrahydro-(2S)-2-naphthoate, methyl 8-methoxy-1,2,3,4-tetrahydro-(2S)-2-naphthoate and methyl 5-methoxy-1,2,3,4-tetrahydro-(2S)-2-naphthoate.

**11.** 8-Methoxy-1,2,3,4-tetrahydro-(2R)-2-naphthoic acid.

**12.** 8-Methoxy-1,2,3,4-tetrahydro-(2S)-2-naphthoic acid.